# EUROPEAN PATENT SPECIFICATION

(11) **EP 2 552 911 B1**
(45) Date of publication and mention of the grant of the patent: **20.07.2016**
(21) Application number: 10769090.1
(22) Date of filing: 28.06.2010
(51) Int. Cl.: C07D 451/10

(54) **TIOTROPIUM BROMIDE PREPARATION PROCESS**
VERFAHREN ZUR HERSTELLUNG VON TIOTROPIUM-BROMID
PROCÉDÉ DE PRÉPARATION DE BROMURE DE TIOTROPIUM

(30) Priority: 01.04.2010 TR 201002520
(43) Date of publication of application: 06.02.2013
(73) Proprietor: Mahmut, Bilgic, Esenler- Istanbul (TR)
(72) Inventor: Mahmut, Bilgic, Esenler- Istanbul (TR)
(86) International application number: PCT/TR2010/000136
(87) International publication number: WO 2011/123077

(56) References cited:
- EP-A1- 0 418 716
- WO-A1-03/057694
- WO-A1-2008/089852
- WO-A1-2009/087419
- HARRY H. WASSERMAN AND BRUCE H. LIPSHUTZ: "Reactions of lithium enolates with molecular oxygen alpha-hydroxylation of amides and other carboxylate derivatives", TETRAHEDRON LETTERS, vol. 16, 1975, pages 1731-1734, XP002614576, DOI: 10.1016/S0040-4039(00)72245-3
- KOSHIMA H ET AL: "Solid-State Photoreaction in Two-Component Molecular Crystals of Thienylacetic Acids and Aza Aromatic Compounds", TETRAHEDRON, ELSEVIER SCIENCE PUBLISHERS, AMSTERDAM, NL, vol. 56, no. 36, 1 September 2000 (2000-09-01), pages 6845-6852, XP004209426, ISSN: 0040-4020, DOI: 10.1016/S0040-4020(00)00506-8

## Description

The present invention relates to a new process for preparing (1α, 2β, 4β, 5α, 7β)-7-[(Hydroxidi-2-thienylacetyl)oxy]-9,9-dimethyl-3-oxa-9-azoniatricyclo[3.3.1.0^{2,4}]nonan-bromide.

### Background of the Invention:

The compound whose chemical name is (1α, 2β, 4β, 5α, 7β)-7-[(Hydroxidi-2-thienylacetyl)oxy]-9,9-dimethyl-3-oxa-9-azoniatricyclo[3.3.1.0^{2,4}]nonan-bromide is generally known as tiotropium bromide. The compound is shown below with formula 5 and was disclosed for the first time in the patent numbered EP418716.

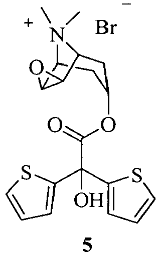

Tiotropium bromide is a highly effective anticholinergic agent and for this reason it is widely used for treatment of asthma and/or COPD (chronic obstructive pulmonary disease).

Tiotropium bromide is generally administered to patients via inhalation. For administration via inhalation, dry powder inhalators wherein the dry powder is filled into blisters/capsules or stored in reservoirs can be used. Another method comprises administration of tiotropium bromide with different gases (e.g. HFA134a and/or HFA227) in aerosol form.

Tiotropyum bromide is a very potent agent and therefore even very small amounts show therapeutic effect.

The patent numbered EP418716 discloses a synthesis method shown in scheme 1 for preparation of tiotropium bromide.

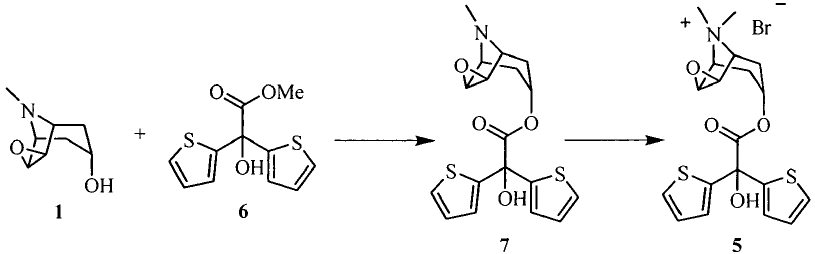

### Scheme 1:

According to this method in the first step scopine that is shown with formula 1 is converted into (2-thienyl)-glycolic acid scopine ester shown with formula 7 by reacting with di-(2-thienyl)-glycolic acid methyl ester that is shown with formula 6. Afterwards compound of formula 7 is quaternized to give tiotropium bromide.

The first step of this synthesis method is carried out at high temperatures like 70-90 °C and in presence of dangerous chemicals like sodium methoxide and metallic sodium. The fact that the process is carried out at high temperatures increases the cost and makes the process undesirable for the producers. Furthermore although the reactions are carried out under harsh conditions the yields are in the range of 45% and 70% and all of these reasons shows that different synthesis methods with higher yields are necessary for preparation of tiotropium bromide.

The patent numbered WO2009/087419 discloses discloses a method for preparation of tiotropium bromide by reacting scopine HCl and methyl di(2-thienyl)glycolate in the presence of a strong base such as sodium hydride an organic heterocyclic amine base, namely 1,8-diazabicyclo[5.4.0]undec-7-ene (DBU). Sodium hydride is insoluble in organic solvents hence when used with organic solvents leads to heterogeneous reactions taking place at the surface of NaH.

The patent numbered WO 03/057694 discloses a synthesis method which comprises use of carbonylimidazole, Lithium imidazole and N-methylpyrrolidone as reagents.

The patent numbered WO 2008/089852 discloses a method for preparation of tiotropium bromide by using methyl scopinium hexaflurorophosphate and methyl di(2-thienyl)glycolate. The document teaches drying the starting materials with molecular sieves prior to addition of LiBr and that use of a strong base such as potassium ter-butoxide while drying the starting materials with molecular sieves increases the yield.

### Detailed description of the invention

Surprisingly, it was found that, compared to the methods present in prior art, tiotropium bromide was obtained under milder conditions and with higher yields when the synthesis method according to present invention was used. In one aspect, the synthesis method comprises use of the method shown in scheme 2.

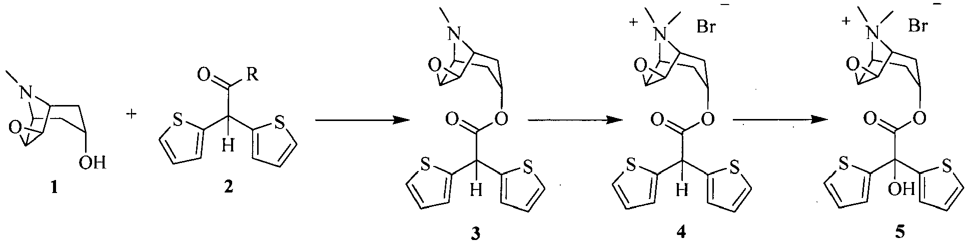

### Scheme 2:

Upon reaction of scopine or acid addition salts thereof with compound of formula 2, compound of formula 3 is obtained. Formula 3 is then converted to compound of formula 4 by quaternization. Afterwards an oxidation reaction carried out under appropriate conditions converts compound of formula 4 into the final compound shown with formula 5 which is tiotropium bromide.

Accordingly, the invention provides a synthesis method for preparation of tiotropium bromide (Formula 5); 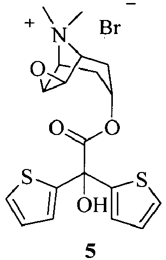 characterized in that; 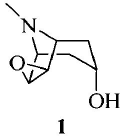 in the first step scopine (Formula 1) or a acid addition salt thereof is reacted with compound shown with Formula 2 wherein R is hydroxy, to give scopine ester shown with Formula 3 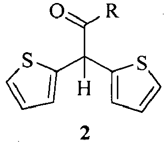 And in the second step, compound shown with Formula 3 is quaternized to give compound of Formula 4

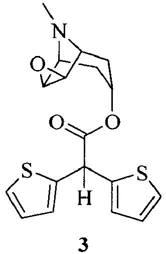

And in the third step compound of Formula 4 is oxidized to give tiotropium bromide.

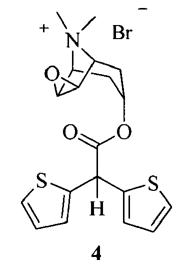

Another aspect of the present invention is use of scopine ester shown with formula 3 in free base form or in form of its acid addition salts for synthesis of tiotropium bromide (5)

In another aspect, another important point of the invention is use of scopine ester shown with formula 3 in free form or when necessary in the form of acid addition salts for the preparation of quaternized scopine ester shown with formula 4. If scopine ester (3) is used in the form of acid addition salts for producing quartenized scopine ester, this salt can be selected from a group comprising hydrochloride, hydrobromide, hydrogenphosphate, hydrogen sulfate, tetrafluoroborate, hexafluorophosphate.

Another aspect of the present invention is related to use of quaternized scopine ester shown with formula 4 for the synthesis of tiotropium bromide (5).

In another aspect, present invention is related to a process for preparation of tiotropium bromide characterized in that; in the first step scopine or an acid addition salt thereof, 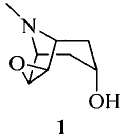 is reacted with compound of formula 2 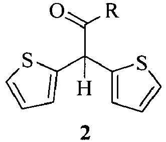 wherein, R group in said compound is hydroxy, to give scopine ester shown with formula 3 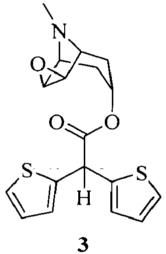 and in the second step, compound of formula 3 is quaternized in presence of methyl bromide to give compound of formula 4 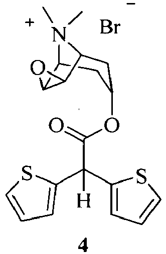 and in the third step compound of formula 4 is subjected to an oxidation reaction in presence of organic/inorganic basic compounds in oxygen contaning atmosphere to give tiotropium bromide.

Compound of formula 2 which can be used to prepare scopine ester of formula 3 is a compound where R is hydroxy.

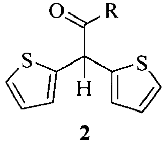

Due to importance of the use of compound of formula 2 in the synthesis of tiotropium bromide as a starting material, another aspect of the present invention relates to use of compound of formula 2 as a starting material for the preparation of tiotropium bromide (5). In accordance with the present invention, scopine (1) can be used in free form or in the form of its acid addition salts such as hydrochloride, hydrobromide, hydrogenphosphate, hydrogensulfate, tetrafluoroborate and hexafluorophosphate. Preferably it is used in free form.

Accordingly a method for preparation of scopine ester shown with formula 3 comprises dissolving scopine in an organic solvent, for example; dimethylformamide (DMF), dimethyl sulfoxide (DMSO), benzene, toluene, diethyl ether, tetrahydrofuran, ethanol, methanol, acetonitrile, acetone, ethyl acetate, methyl ethyl ketone, dichloromethane, dioxane, dimethylacetamide, N-methyl pyrrolidone, hexane, heptane or a mixture of these solvents, preferably dimethylformamide or dichloromethane is used.

If scopine is used in the form of its acid addition salt then a base is added to said solution in order to liberate scopine. According to present invention the base is selected from a group consisting of organic or inorganic bases, for example; from a group comprising triethylamine, diisopropylethylamine, pyridine, dimethylaminopyridine, N-methylpyrolidine, N-methylmorpholine or ammonia. Preferably ammonia is used. Said basic compound is used in an amount of at least 1 mole, preferably in an amount of 1.25 to 2.5 moles, more preferably in an amount of 1.5 to 2.0 moles per 1 mole of scopine used. Addition of the basic compound can take place at a temperature of 0 to 60 °C, preferably 15 to 50 °C, and more preferably 20 to 30 °C. Afterwards the obtained mixture is stirred for 0.5 to 3 hours, more preferably for 1 to 2 hours at a fixed temperature. The salt that forms during the reaction is separated by filtration and the solvent present in the obtained solution is distilled under appropriate heat and pressure. These conditions are determined according to the nature of the solvent used.

The compound obtained is then dissolved in an appropriate organic solvent, for example, dimethylformamide (DMF), dimethyl sulfoxide (DMSO), benzene, toluene, diethyl ether, tetrahydrofuran, ethanol, methanol, acetonitrile, acetone, ethyl acetate, methyl ethyl ketone, dichloromethane, dioxane, dimethylacetamide, N-methyl pyrrolidone, hexane, heptane or a mixture thereof and compound of formula 2 is added to the formed solution.

The compound of formula 2 used in this process is selected from compounds wherein R is hydroxy.

In an embodiment not of the claimed invention, the R group in formula 2 may be methoxy, ethoxy, vinyloxy, phenyloxy, -S-methyl, -S-ethyl or -S-phenyl - with these R substituents, the reaction is carried out in presence of an organic or inorganic base. As organic base alkali carbonates, or earth alkali carbonates; lithium carbonate, sodium carbonate, potassium carbonate, calcium carbonate, sodium hydride, potassium hydride, calcium hydride, sodium methylate, sodium ethylate, potassium methylate or potassium ethylate can be used. As inorganic base one of the hydrides mentioned above is used. Preferably sodium hydride is used. Said basic compound is added at least in stochiometric amount, preferably in 1 to 3 moles, more preferably 1.5 to 2 moles per mole of scopine. The solution obtained after addition of the base is stirred for 10-120 minutes preferably for 30-90 minutes. If the compound of formula 2 is an ester wherein R is methoxy or ethoxy then the reaction is carried out at a temperature of 40-90 °C preferably at 50-80 °C and more preferably at 60-75 °C, under vacuum in order to distill off the alcohol that forms as a side product and thus shift the reaction to the side of the scopine ester.

After completion of the distillation, if necessary, solvent that was also removed during the distillation of the side product can be added again. The obtained solution is then cooled to -5 to 40 °C, preferably to 0-35 °C, more preferably to 10-25 °C. And then hydrochloric acid is added to said solution over 12-120 minutes, preferably over 25-50 minutes. Hydrochloric acid used in this step can be in gaseous form or in form of an aqueous solution; preferably aqueous solution of hydrochloric acid is used. Per one mole of scopine, 80-350 ml, preferably 120-225 ml of 36% hydrochloric acid that is dissolved in 10-20 liters, preferably 12-17 liters of water is added.

After addition of hydrochloric acid solution is complete, the aqueous phase is separated and washed with a water immiscible organic solvent, for example methylene chloride, ethylacetate, toluene, n-butyl acetate, preferably with dichloromethane and then the organic layer is separated and discarded. This step can be repeated if necessary.

The aqueous phase that is obtained is combined with a water immiscible organic solvent, for example; methylene chloride, ethylacetate, toluene, n-butylacetate and an inorganic base, for example carbonates of alkali metals or alkali earth metals for example; lithium carbonate, sodium carbonate, potassium carbonate, calcium carbonate and preferably sodium carbonate is added and pH of the solution is adjusted to 7.5-11, preferably 8-10. Inorganic base is preferably added in the form of its aqueous solution. Accordingly, per mole of scopine, 50-400 g, preferably 100-200 g inorganic base is used by dissolving in 0.25-1.5 L, preferably in 0.5-1.0 L, most preferably in 0.7-0.8 L of water.

After thoroughly mixing the obtained mixture, organic layer is separated. Aqueous phase is washed with a water immiscible organic solvent, for example methylene chloride, ethyl acetate, toluene, n-butylacetate, if necessary this step can be repeated one more time, afterwards organic layers are combined and the organic solvent is removed under appropriate heat and pressure.

The compound obtained after distillation is dissolved in an appropriate solvent, for example dimethylformamide (DMF), dimethyl sulfoxide (DMSO), benzene, toluene, diethyl ether, tetrahydrofuran, ethanol, methanol, acetonitrile, acetone, ethyl acetate, methyl ethyl ketone, dichloromethane, dioxane, dimethylacetamide, N-methyl pyrrolidone, hexane, heptane and a mixture thereof and the obtained solution is heated to the boiling point of the solvent and afterwards slowly cooled to a temperature between -10 to 20 °C. Scopine ester that is obtained as a result of this solution is separated by filtration and dried under vacuum.

With the R group that is in the compound shown with formula 2 as hydroxy, this compound may also be shown with formula 2a;

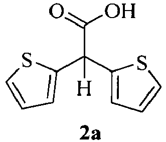

The synthesis method for obtaining scopine ester shown with formula 3 comprises the steps of; preparing scopine or an acid addition salt thereof as described before and then dissolving said compound in an appropriate solvent, for example; dimethylformamide (DMF), dimethyl sulfoxide (DMSO), benzene, toluene, diethylether, tetrahydrofuran, ethanol, methanol, acetonitrile, acetone, ethyl acetate, methyl ethyl ketone, dichloromethane, dioxane, dimethylacetamide, N-methyl pyrrolidone, hexane, heptane or a mixture thereof and addition of compound of formula 2 wherein R group is hydroxy to said solution and afterwards adding N,N-dicyclohexylcarbodiimide (DCC) 1-ethyl-3-(3-dimethylaminopropyl)carbodiimide (EDC) or N,N'-diisopropylcarbodiimide and optionally 4-dimethylaminopyridine (DMAP). Said reaction mixture is stirred at a temperature of 0-40 °C, preferably at room temperature for 10-48, preferably for 12-18 hours.

In this reaction per one mole of scopine, at least 1 mole, preferably 1.05-3 moles, more preferably 1.1-1.8 moles of DCC, EDC or DCI is used. Optionally, per one mole of scopine at least 0.01 moles, preferably 0.03 to 0.2 moles, more preferably 0.05-0.1 moles of DMAP can be added.

At the end of the reaction if solid particles form, the solution is filtered to remove these solid particles, then the solution obtained is diluted with an appropriate organic solvent, for example; dichloromethane, ethyl acetate, hexane, heptanes, and then extracted with water. After removal of organic phase from water phase, the solvent that is used is removed under appropriate conditions. At this point the conditions, e.g. the temperature and pressure, are determined according to the nature of the solvent that is used.

The obtained compound can be purified with conventional purification methods; for example, crystallization with anti-solvent, crystallization with active carbon, thin layer chromatography (TLC), column chromatography, high pressure liquid chromatography (HPLC), distillation etc. if need be.

A method for preparation of quaternized scopine ester shown with formula 4 comprises, dissolving scopine ester shown with formula 3 and/or acid addition salts thereof and a methyl bromide solution comprising methyl bromide in an amount 10-90 %, preferably 30-60% by weight in a suitable organic solvent; for example dimethylformamide (DMF), dimethyl sulfoxide (DMSO), benzene, toluene, diethylether, tetrahydrofuran, ethanol, methanol, acetonitrile, acetone, ethyl acetate, methyl ethyl ketone, dichloromethane, dioxane, dimethylacetamide, N-methyl pyrrolidone, hexane, heptane or a mixture thereof and stirring the formed mixture at a temperature of 0-40 °C, preferably at room temperature for 12-90 hours, preferably for 18-72 hours.

Methyl bromide solution is prepared by condensing methyl bromide gas at low temperature and then mixing the obtained methyl bromide with an organic solvent; for example, dimethylformamide (DMF), dimethyl sulfoxide (DMSO), benzene, toluene, diethyl ether, tetrahydrofuran, ethanol, methanol, acetonitrile, acetone, ethyl acetate, methyl ethyl ketone, dichloromethane, dioxane, dimethylacetamide, N-methyl pyrrolidone, hexane, heptane or a mixture thereof. The solution is preferably prepared in acetonitrile.

The compound that is obtained can be purified by one of the conventional purification methods for example; crystallization by anti-solvent, crystallization by use of active carbon, thin layer chromatography (TLC), column chromatography, high pressure liquid chromatography (HPLC), distillation etc.

A process for preparation of tiotropium bromide comprises, dissolving quaternized scopine ester compound shown with formula 4 in an organic solvent, for example dimethylformamide (DMF), dimethyl sulfoxide (DMSO), benzene, toluene, diethyl ether, tetrahydrofuran, ethanol, methanol, acetonitrile, acetone, ethyl acetate, methyl ethyl ketone, dichloromethane, dioxane, dimethylacetamide, N-methyl pyrrolidone, hexane, heptane or a mixture thereof that is saturated with oxygen. Preferably acetonitrile or dichloromethane that is saturated with oxygen is used. Then an inorganic base for example; potassium carbonate, sodium carbonate, potassium hydrogen carbonate, sodium hydrogen carbonate, sodium tert-butoxide, triethylamine is added to the formed solution, the formed mixture is stirred at a temperature of -78 to 70 °C, preferably at -30 to 60 °C, for 1-72 hours, preferably for 3-48 hours.

The compound that is obtained can be purified by one of the conventional purification methods for example; crystallization by anti-solvent, crystallization by use of active carbon, thin layer chromatography (TLC), column chromatography, high pressure liquid chromatography (HPLC), distillation etc.

The present invention also relates to the use of intermediates for the preparation of tiotropium bromide, namely; scopine ester shown with formula 3 and its acid addition salts, for example hydrochloride, hydrobromide, hydrogenphosphate, hydrogen sulphate, tetrafluoroborate, hexafluorophosphate salts 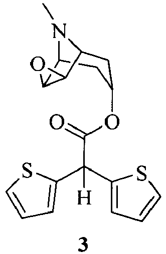 And quaternized scopine ester shown with formula 4,

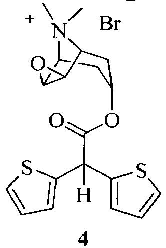

The present invention also relates to, pharmaceutical compositions comprising tiotropium bromide prepared according to the present invention and use of said pharmaceutical compositions for the treatment of pulmonary diseases such as asthma, chronic obstructive pulmonary disease (COPD) and allergic rhinitis.

The examples below are given to explain the subject-matter synthesis method, and the present invention should not be limited with these examples.

### Example 1: A process for preparation of scopine ester (3)

Scopine (155.2 g, 1 mole) and di-(2-thienyl) acetic acid (246.4 g, 1.1 moles) are dissolved in dichlorometane (700 ml). DCC (268.2 g, 1.3 moles) and DMAP (49 g, 0.4 moles) are then added to the obtained solution and the mixture is then stirred at room temperature for 16 hours. The solid by-product is filtered off and the obtained clear solution is concentrated under low pressure. The crude product is then purified by column chromatography to give 274.4 g of scopine ester (3) (76% yield).

### Example 2: A process for preparation of scopine ester (3)

Scopine (7.8 g, 0.05 moles) and di-(2-thienyl) acetic acid (12.3 g, 0.055 moles) are dissolved in dichloromethane (80 ml). DCC (15.5 g, 0.075 moles) is then added to the obtained solution and the mixture is stirred at room temperature for 16 hours. The solid by-product is filtered off. The solution is then diluted with dichloromethane (20 ml) and extracted with distilled water (50 ml x 3). The organic layer is separated and dried with anhydrous Na₂SO₄ and filtered. The obtained clear solution is concentrated under low pressure. The crude product is then purified by column chromatography to give 14.4 g of scopine ester (3) (80% yield)

### Example 3: A process for preparation of scopine ester (3)

Scopine (11.6 g, 0.075 moles) and di-(2-thienyl) acetic acid (20.2 g, 0.09 moles) are dissolved in dimethylformamide (50 ml). EDC (14.0 g, 0.09 moles) is then added to the obtained solution and the mixture is stirred for 16 hours at room temperature. The organic solvent is then removed under reduced pressure at a temperature of 50 °C. The obtained product is then diluted with dichloromethane (100 ml) and extracted with distilled water (100 ml x 3). The organic phase obtained at the end of the extraction process is separated and then dried with anhydrous Na₂SO₄ and filtered. The organic solvent is then removed under reduced pressure to give 19.5 g of scopine ester (3) (72% yield).

### Example 4: A process for preparation of scopine ester (3)

Scopine (15.5 g, 0.1 moles) and di-(2-thienyl) acetic acid (24.6 g, 0.11 moles) are dissolved in dichloromethane (100 ml). EDC (17.1 g, 0.11 moles) and DMAP (1.2 g, 0.01 moles) are then added to the obtained solution and the mixture is stirred at room temperature for 16 hours. The obtained product is extracted with distilled water (50 ml x 3). At the end of extraction process organic phase is separated and dried with anhydrous Na₂SO₄ and then filtered. Organic solvent is then removed under reduced pressure to give 30.0 g of scopine ester (3) (83% yield).

### Example 5: A process for preparation of scopine ester (3)

Scopine (1.4 g, 0.009 moles) and di-(2-thienyl) acetic acid (2.2 g, 0.010 moles) are dissolved in dichloromethane (20 ml). DCC (2.04 g, 0.010 moles) is then added to the obtained solution and the mixture is stirred at room temperature for 16 hours. The solid by-product is removed by filtration. The organic solvent is removed under reduced pressure. The crude product is then purified by column chromatography to give 1.95 g of scopine ester (3) (60 % yield).

### Example 6: A process for preparation of quaternized scopine ester (4)

Scopine ester (3) (0.8 g, 0.0022 moles) and and acetonitrile solution of methyl bromide (50% wt/wt) (1 ml) are dissolved in acetonitrile and the mixture is the stirred at room temperature for 72 hours. The solid precipitate is then filtered, washed with a suitable solvent and dried under low pressure to give 941 mg of quaternized scopine ester (4) (94% yield).

### Example 7: Process for preparation of quaternized scopine ester (4)

Scopine ester (3) (0.8 g, 0.0022 moles) and acetonitrile solution of methyl bromide (50% wt/wt) (1 ml) is dissolved in acetonitrile (1 ml) and the obtained mixture is stirred at room temperature for 24 hours. The formed solid precipitate is then filtered and washed with a suitable solvent, e.g. acetonitrile and dried under vacuum to give 981 mg of quaternized scopine ester (4) (98% yield).

### Example 8: Process for preparation of Tiotropium bromide (5)

Quaternized scopine ester **(4)** (0.6 g, 1.32 mmol) and triethylamine (0.36 mL) are dissolved in acetonitrile (12 mL) that is saturated with oxygen gas. The obtained mixture is then stirred at room temperature for 48 hours. The precipitate that forms is then filtrated and the solid is washed with a suitable solvent e.g. acetonitrile, and dried under vacuum to give 460 mg of tiotropium bromide (74% yield).

## Claims

1. A synthesis method for preparation of tiotropium bromide (Formula 5); 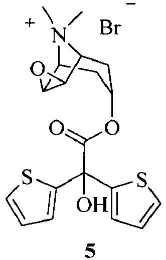 **characterized in that**; in the first step scopine (Formula 1) or a acid addition salt thereof 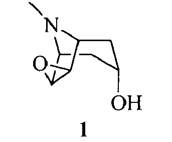 is reacted with compound shown with Formula 2 wherein R is hydroxy, to give scopine ester shown with Formula 3 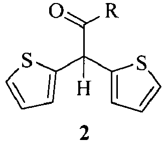 And in the second step, compound shown with Formula 3 is quaternized to give compound of Formula 4 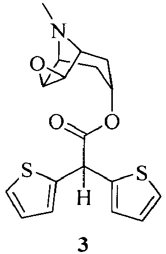 And in the third step compound of Formula 4 is oxidized to give tiotropium bromide.

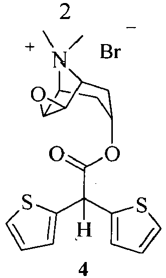

2. A synthesis method according to claim 1, wherein the process for preparation of scopine ester shown with Formula 3 comprises dissolving scopine (1) or an acid addition salt thereof and compound shown with Formula 2 and suitable reagents in an organic solvent.

3. A process for preparation of scopine ester shown with Formula 3 according to claim 2, wherein the process comprises the steps of dissolving scopine and compound of Formula 2 in a suitable organic solvent and then adding N,N'-dicyclohexylcarbodiimide (DCC), 1-ethyl-3-(3-dimethylaminopropyl)carbodiimide (EDC) or N,N'-diisopropylcarbodiimide (DIC), preferably DCC or EDC, most preferably DCC and optionally 4-dimethylaminopyridine (DMAP) to the formed solution.

4. A process for preparation of scopine ester shown with Formula 3 according to claim 3, wherein at least 1 mole of DCC, EDC or DIC is added to the reaction mixture per mole of scopine.

5. A process for preparation of scopine ester according to claim 3, wherein in said reaction optionally at least 0.01 molof DMAP is added per mole of scopine.

6. A synthesis method according to claim 1, wherein a process for preparation of quaternized scopine ester shown with Formula 4 comprises the steps of dissolving scopine ester shown with Formula 3 and/or an acid addition salt thereof and 10-90%, methyl bromide solution in a suitable organic solvent.

7. A process for preparation of compound of Formula 4 according to claim 6, wherein scopine ester is used in free form or in the form of its acid addition salts wherein said acid addition salt is selected from a group comprising hydrochloride, hydrobromide, hydrogenphosphate, hydrogen sulphate, tetrafluoroborate, hexafluorophosphate.

8. A synthesis method according to claim 1, wherein the process for preparation of tiotropium bromide shown with Formula 5, comprises the steps of dissolving quaternized scopine ester shown with Formula 4 in an organic solvent saturated with oxygen and addition of an organic or inorganic base to the obtained solution.

9. Use of a compound of Formula 2a for synthesis of tiotropium bromide as described in claim 1.

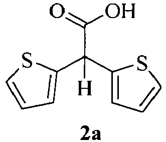

10. Use of a compound shown with Formula 3, wherein said compound can be in free base form or in the form of its acid addition salts, such as hydrochloride, hydrobromide, hydrogenphosphate, hydrogen sulphate, tetrafluoroborate, hexafluorophosphatefor synthesis of tiotropium bromide as described in claim 1.

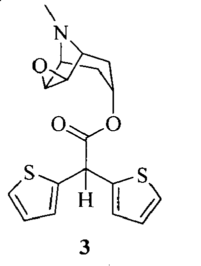

11. Use of a compound shown with Formula 4 for synthesis of tiotropium bromide as described in claim 1.

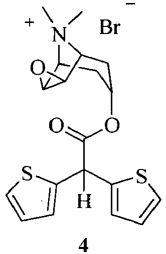

## Patentansprüche

1. Ein Syntheseverfahren zur Herstellung von Tiotropiumbromid (Formel 5); 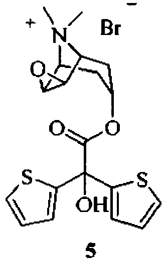 **dadurch gekennzeichnet, dass** im ersten Schritt Scopin- (Formel 1) oder ein Säureadditionssalz davon 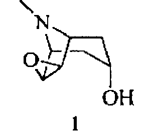 it der Verbindung gezeigt mit der Formel 2 reagiert wird, wobei R Hydroxy ist, Scopinester, der mit der Formel 3 gezeigt wird, zu geben 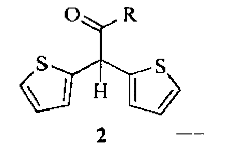 Und im zweiten Schritt Verbindung, die mit der Formel 3 gezeigt wird, quaternisiert wird, die Verbindung der Formel 4 zu geben, 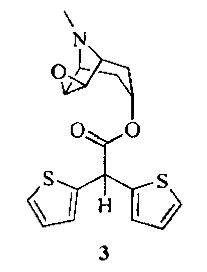 Und im dritten Schritt Verbindung der Formel 4 oxidiert wird, Tiotropiumbromid zu geben.

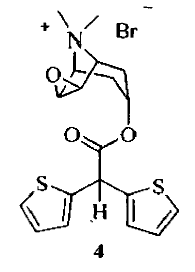

2. Ein Syntheseverfahren nach Anspruch 1, wobei das Verfahren zur Herstellung von Scopinester, der mit der Formel 3 gezeigt wird, auflösendes Scopin (1) oder ein Säureadditionssalz davon und die Verbindung, die mit der Formel 2 gezeigt wird, und geeignete Reagenzien in einem organischen Lösungsmittel umfasst.

3. Ein Verfahren zur Herstellung von Scopinester, der mit der Formel 3 gezeigt wird, nach Anspruch 2, wobei das Verfahren die Schritte des auflösenden Scopin und Verbindung der Formel 2 in einem geeigneten organischen Lösungsmittel und dann Zugabe von N, N 'Dicyclohexylcarbodiimid (DCC), 1-Ethyl -3- (3-dimethylaminopropyl) carbodiimid (EDC) oder N, N'-Diisopropylcarbodiimid (DIC), vorzugsweise DCC oder EDC, am meisten bevorzugt DCC und wahlweise 4-Dimethylaminopyridin (DMAP) zu der gebildeten Lösung umfasst.

4. Ein Verfahren zur Herstellung von Scopinester, der mit der Formel 3 gezeigt wird, nach Anspruch 3, wobei mindestens 1 Mol von DCC, EDC oder DIC zu dem Reaktionsgemisch pro Mol des Scopins zugegeben wird.

5. Ein Verfahren zur Herstellung von Scopinester nach Anspruch 3, wobei in der genannten Reaktion gegebenenfalls mindestens 0,01 Mol von DMAP pro Mol des Scopins zugegeben wird.

6. Ein Syntheseverfahren nach Anspruch 1, wobei ein Verfahren zur Herstellung vom quaternisierten Scopinester, der mit der Formel 4 gezeigt wird, die Schritte des auflösenden Scopinester, der mit der Formel 3 gezeigt wird, und/oder ein Säureadditionssalz davon und 10-90% von Methylbromid-Lösung in einem geeigneten organischen Lösungsmittel umfasst.

7. Ein Verfahren zur Herstellung der Verbindung Formel 4 nach Anspruch 6, wobei Scopinester in freier Form oder in der Form seiner Säureadditionssalze verwendet wird, wobei das genannte Säureadditionssalz aus einer Gruppe enthaltend Hydrochlorid , Hydrobromid, Hydrogenphosphat , Hydrogensulfat , Tetrafluoroborat und Hexafluorophosphat ausgewählt wird.

8. Ein Syntheseverfahren nach Anspruch 1, wobei das Verfahren zur Herstellung von Tiotropiumbromid, das mit der Formel 5 gezeigt wird, die Schritte des auflösenden quaternisierten Scopinester, der mit der Formel 4 in einem organischen Lösungsmittel gezeigt wird, das mit Sauerstoff gesättigt wird, und Zugabe einer organischen oder anorganischen Base zu der erhaltenen Lösung umfasst.

9. Verwendung einer Verbindung der Formel 2a für Synthese von Tiotropiumbromid nach Anspruch 1.

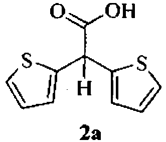

10. Verwendung einer Verbindung, die mit der Formel 3 gezeigt wird, wobei die genannte Verbindung in freier Form oder in der Form ihrer Säureadditionssalze wie Hydrochlorid, Hydrobromid, Hydrogenphosphat , Hydrogensulfat, Tetrafluoroborat, hexafluorophosphatefor-Synthese von Tiotropiumbromid nach Anspruch 1 sein kann.

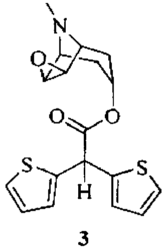

11. Verwendung einer Verbindung, die mit der Formel 4 gezeigt wird, für Synthese von Tiotropiumbromid nach Anspruch 1.

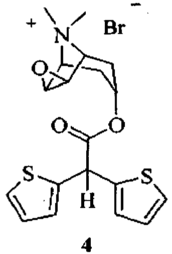

## Revendications

1. La méthode de synthèse pour la préparation de bromide de tiotropium (Formule 5); 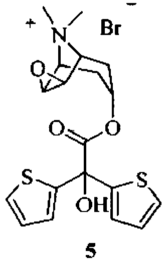 Est caractérisée dans, dans la première étape de scopine (Formule 1) ou un sel d'acide en addition de celui-ci 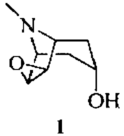 Est reacteé avec la composée représenté par la Formule 2 dans laquelle R est hydroxyl , pour donner l'ester de scopine par la Formule 3 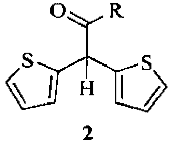 Et dans la deuxième étape, le composé representé par la Formule 3 est quaternisé pour donner un composé de Formule 4 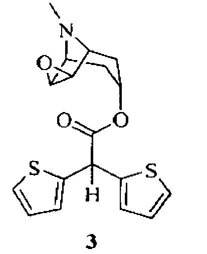 Et dans la troisième étape le composé de Formule 4 est oxydé pour donner bromide de tiotropium.

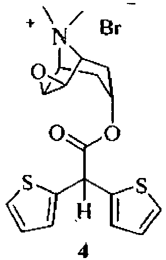

2. Un méthode de synthèse selon la revendication 1, dans lequel le procédé de préparation d'un ester de scopine représenté par la Formule 3 comprend la dissolution de scopine (1) ou un sel d'addition d'acide de celui-ci et un composé représenté par la Formule 2 et des réactifs appropriés dans un solvant organique.

3. Un procédé de préparation d'un ester de de scopine représenté par la formule 3 selon la revendication 2, dans lequel le procédé comprend les étapes de dissolution de scopine et le composé de formule 2 dans un solvant organique approprié et ensuite en ajoutant du N, N'-dicyclohexylcarbodiimide (DCC), 1- éthyl-3- (3-diméthylaminopropyl) carbodiimide (EDC) ou le N, N'-diisopropylcarbodiimide (DIC), de préférence le DCC ou l'EDC, le DCC le plus préférablement et optionnellement de 4-diméthylaminopyridine (DMAP) à la solution formée.

4. Un procédé de préparation d'un ester de scopine représenté par la formule 3 selon la revendication 3, dans lequel au moins 1 mole de DCC, EDC ou DIC est ajouté au mélange réactionnel par mole de de scopine.

5. Un procédé de préparation d'un ester de de scopine selon la revendication 3, dans lequel, dans ladite réaction, optionnellement d'au moins 0,01 mole of DMAP est ajouté par mole de scopine.

6. Un méthode de synthèse selon la revendication 1, dans lequel un procédé de préparation d'un ester de scopine quaternisé représenté par la formule 4 comprend les étapes consistant dissolution d'ester de scopine représenté par la formule 3 et / ou un sel d'addition d'acide de celui-ci et de 10 à 90%, une solution de bromure de méthyle dans un solvant organique approprié.

7. Un procédé de préparation d'un composé de Formule 4 selon la revendication 6, dans lequel l'ester de scopine est utilisé sous forme libre ou sous forme de ses sels d'addition d'acides, dans lequel ledit sel d'addition d'acide est choisi dans un groupe comprenant le chlorhydrate, le bromhydrate, l'hydrogène phosphate, hydrogène sulfate , tétrafluoroborate, hexafluorophosphate.

8. Un méthode de synthèse selon la revendication 1, dans lequel le procédé de préparation du bromure de tiotropium représenté par la formule 5, comprenant les étapes consistant à dissoudre l'ester de scopine quaternisé représenté par la formule 4 dans un solvant organique saturé avec l'oxygène et l'addition d'une base organique ou inorganique à la solution obtenue.

9. Utilisation d'un composé de Formule 2a pour la synthèse du bromure de tiotropium selon la revendication 1. 3

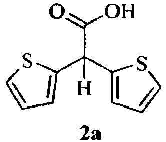

10. Utilisation d'un composé représenté par la formule 3, dans laquelle ledit composé peut être sous forme de base libre ou sous forme de sels d'addition d'acides de celui-ci, comme le chlorhydrate, le bromhydrate, l'hydrogénophosphate, hydrogénosulfate, tétrafluoroborate, hexafluorophosphate pour la synthèse de bromure de tiotropium tel que décrit dans la revendication 1.

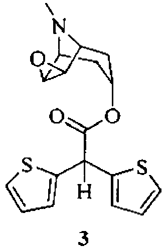

11. Utilisation d'un composé représenté par la Formule 4 pour la synthèse du bromure de tiotropium comme décrit dans la revendication 1.
